# EUROPEAN PATENT APPLICATION

(11) **EP 1 439 234 A1**
(43) Date of publication of application: **21.07.2004**
(21) Application number: 03000249.7
(22) Date of filing: 08.01.2003
(51) Int. Cl.: C12N 15/90, C12N 15/63, C12N 5/10, A01K 67/00

(54) **Targeted transgenesis using the rosa26 locus**

(71) Applicant: ARTEMIS Pharmaceuticals GmbH, 51063 Köln (DE)
(72) Inventor: Schwenk, Frieder, 50999 Köln (DE); Seibler, Jost, 50733 Köln (DE); Faust, Nicole, 51503 Rösrath (DE); Kühn, Ralf, 50937 Köln (DE)
(74) Representative: Helbing, Jörg, Dr. Dipl.-Chem.

(57) **Abstract**

The invention provides a method for targeted transgenesis using the Rosa26 locus. Suitable nucleotide acid sequences and vectors for the targeted transgenesis are provided. The Rosa26 locus proved to be a suitable integration site allowing strong and predictable expression of inserted transgenes carrying exogenous promoters.

SEQUENCE LISTING

<110> ARTEMIS Pharmaceutical GmbH

<120> Targeted transgenesis using the Rosa26 locus

<130> 023403ep JH/BM

<140>
<141>

<160> 7

<170> PatentIn Ver. 2.1

<210> 1
<211> 23
<212> DNA
<213> Artificial Sequence

<220>
<223> Description of Artificial Sequence: primer
      Rscreen1s

<400> 1
gacaggacag tgcttgttta agg                                         23

<210> 2
<211> 23
<212> DNA
<213> Artificial Sequence

<220>
<223> Description of Artificial Sequence: primer
      Rscreen1as

<400> 2
tgactacaca atattgctcg cac                                         23

<210> 3
<211> 13139
<212> DNA
<213> Mus musculus

<220>
<223> Description: Rosa26 locus

<400> 3
aagcttctca cgtagcaacc agagctccag agccagcagc tgctgccgcc ttgtatactc 60
actcctgtga tccaacacag gagcaacctt ttctttaccc cacccccact tcttaacaca 120
cttttttttg gggggggggg gggaacaagt gctccatgct ggaaggattg gaactatgct 180
tttagaaagg aacaatccta aggtcacttt taaattgagg tctttgattt gaaaatcaac 240
aaataccaaa ttccaaatat tcgttttaat taaaccagca atgtggatat aagcattaag 300
ttttagtttt aaaaaggtca attttccaaa cattcagcaa tcatatttaa atttacagct 360
aggaacaaga gccttgggtc atgtcctacc aaagaacata actcaatatt ctacacatga 420
caatctgaat aaccttaaag cctctaatcc cataacaggc cacaaatttt ggacagagaa 480
ctaatgatcc tcctgagaaa actggaagaa atccagggaa aagaaattcc tgtgtcctcc 540
aaactcagaa atctctaatt atgtcagtat tctctgcttt agtcctaggt cagattgcac 600
acatctaaaa taacctctta aagttttcct cctagcgacc taaaccatta ttaatatcaa 660

attaaccatc aaaacacttt cctctcaata tgctgcacac aaacctcctc ctggaacctc 720
ctccatctgg atcctcccca atcaaaagta taggtattta acatataagc aaggaagtaa 780
tgtaaacatg accttggtca caaatatgtc atctaaaaac aatttagtca aggtatggag 840
gaaattcgag aacctgaatc tttttaagta ttttgagcac aggaacaatt ggcaaaagga 900
atccaggtat agacaaaacc cagagcccag agctctgggc gaaaaatgag ttgctggtga 960
agacgttaca caagtaacat gagaaagcag aaaatgcagg tcatccacgc acccctgacc 1020
caggccagca gggcgggctg cagcatcagt acacaggaga aagatcctta ttcctaagaa 1080
tgagaaaggc aaaggcgccc gatagaataa attagcatag aaggggcttt cccaggagtt 1140
aaaactttcc ttctgagcga ttacctacta aaaccagggc ttttgcccac taccatttac 1200
ctaggatctt ggcttgcacg gattcatagg ggcatatccc tccccctctt ctttagagtc 1260
gttcttaaaa gatcgctctc cacgccctag gcagggaaaa cgacaaaatc tggctcaatt 1320
ccaggctaga accctacaaa ttcaacaggg atatcgcaag gatactgggg catacgccac 1380
agggagtcca agaatgtgag gtgggggtgg cgaaggtaat gtctttggtg tgggaaaagc 1440
agcagccatc tgagatagga actggaaaac cagaggagag gcgttcagga agattatgga 1500
ggggaggact gggcccccac gagcgaccag agttgtcaca aggccgcaag aacaggggag 1560
gtggggggct cagggacaga aaaaaaagta tgtgtatttt gagagcaggg ttgggaggcc 1620
tctcctgaaa agggtataaa cgtggagtag gcaataccca ggcaaaaagg ggagaccaga 1680
gtagggggag gggaagagtc ctgacccagg gaagacatta aaaaggtagt ggggtcgact 1740
agatgaagga gagcctttct ctctgggcaa gagcggtgca atggtgtgta aaggtagctg 1800
agaagacgaa aagggcaagc atcttcctgc taccaggctg gggaggccca ggcccacgac 1860
cccgaggaga gggaacgcag ggagactgag gtgacccttc tttcccccgg ggcccggtcg 1920
tgtggttcgg tgtctctttt ctgttggacc cttaccttga cccaggcgct gccggggcct 1980
gggcccgggc tgcggcgcac ggcactcccg ggaggcagcg agactcgagt taggcccaac 2040
gcggcgccac ggcgtttcct ggccgggaat ggcccgtacc cgtgaggtgg gggtgggggg 2100
cagaaaaggc ggagcgagcc cgagcgggga gggggagggc caggggcgga gggggccggc 2160
actactgtgt tggcggactg gcgggactag ggctgcgtga gtctctgagc gcaggcgggc 2220
ggcggccgcc cctcccccgg cggcggcagc ggcggcagcg gcggcagctc actcagcccg 2280
ctgcccgagc ggaaacgcca ctgaccgcac ggggattccc agtgccggcg ccaggggcac 2340
gcgggacacg ccccctcccg ccgcgccatt ggcctctccg cccaccgccc cacacttatt 2400
ggccggtgcg ccgccaatca gcggaggctg ccggggccgc ctaaagaaga ggctgtgctt 2460
tggggctccg gctcctcaga gagcctcggc taggtagggg atcgggactc tggcgggagg 2520
gcggcttggt gcgtttgcgg ggatgggcgg ccgcggcagg ccctccgagc gtggtggagc 2580
cgttctgtga gacagccggg tacgagtcgt gacgctggaa ggggcaagcg ggtggtgggc 2640
aggaatgcgg tccgccctgc agcaaccgga gggggaggga gaagggagcg gaaaagtctc 2700
caccggacgc ggccatggct cggggggggg ggggcagcgg aggagcgctt ccggccgacg 2760
tctcgtcgct gattggcttc ttttcctccc gccgtgtgtg aaaacacaaa tggcgtgttt 2820
tggttggcgt aaggcgcctg tcagttaacg gcagccggag tgcgcagccg ccggcagcct 2880
cgctctgccc actgggtggg gcgggaggta ggtggggtga ggcgagctgg acgtgcgggc 2940
gcggtcggcc tctggcgggg cgggggaggg gagggagggt cagcgaaagt agctcgcgcg 3000
cgagcggccg cccaccctcc ccttcctctg ggggagtcgt tttacccgcc gccggccggg 3060
cctcgtcgtc tgattggctc tcggggccca gaaaactggc ccttgccatt ggctcgtgtt 3120
cgtgcaagtt gagtccatcc gccggccagc gggggcggcg aggaggcgct cccaggttcc 3180
ggccctcccc tcggccccgc gccgcagagt ctggccgcgc gcccctgcgc aacgtggcag 3240
gaagcgcgcg ctgggggcgg ggacgggcag tagggctgag cggctgcggg gcgggtgcaa 3300
gcacgtttcc gacttgagtt gcctcaagag gggcgtgctg agccagacct ccatcgcgca 3360
ctccggggag tggagggaag gagcgagggc tcagttgggc tgttttggag gcaggaagca 3420
cttgctctcc caaagtcgct ctgagttgtt atcagtaagg gagctgcagt ggagtaggcg 3480
gggagaaggc cgcacccttc tccggagggg ggaggggagt gttgcaatac ctttctggga 3540
gttctctgct gcctcctggc ttctgaggac cgccctgggc ctgggagaat cccttccccc 3600
tcttccctcg tgatctgcaa ctccagtctt tctagaagat gggcgggagt cttctgggca 3660
ggcttaaagg ctaacctggt gtgtgggcgt tgtcctgcag gggaattgaa caggtgtaaa 3720
attggaggga caagacttcc cacagatttt cggttttgtc gggaagtttt ttaatagggg 3780
caaataagga aaatgggagg ataggtagtc atctggggtt ttatgcagca aaactacagg 3840
ttattattgc ttgtgatccg cctcggagta ttttccatcg aggtagatta aagacatgct 3900
cacccgagtt ttatactctc ctgcttgaga tccttactac agtatgaaat tacagtgtcg 3960
cgagttagac tatgtaagca gaattttaat catttttaaa gagcccagta cttcatatcc 4020
atttctcccg ctccttctgc agccttatca aaaggtattt tagaacactc attttagccc 4080
cattttcatt tattatactg gcttatccaa cccctagaca gagcattggc attttccctt 4140
tcctgatctt agaagtctga tgactcatga aaccagacag attagttaca tacaccacaa 4200
atcgaggctg tagctggggc ctcaacactg cagttctttt ataactcctt agtacacttt 4260
ttgttgatcc tttgccttga tccttaattt tcagtgtcta tcacctctcc cgtcagtggt 4320

gttccacatt tgggcctatt ctcagtccag ggagttttac aacaatagat gtattgagaa 4380
tccaacctaa agcttaactt tccactccca tgaatgcctc tctccttttt ctccatttat 4440
aaactgagct attaaccatt aatggttcca ggtggatgtc tcctccccat attacctgat 4500
gtatcttaca tattgccagg ctgatatttt aagacattaa aaggtatatt tcattattga 4560
gccacatggt attgattact gcttactaaa attttgtcat tgtacacatc tgtaaaaggt 4620
ggttcctttt ggaatgcaaa gttcaggtgt ttgttgtctt tcctgaccta aggtcttgtg 4680
agcttgtatt ttttctattt aagcagtgct ttctcttgga ctggcttgac tcatggcatt 4740
ctacacgtta ttgctggtct aaatgtgatt ttgccaagct tcttcaggac ctataatttt 4800
gcttgacttg tagccaaaca caagtaaaat gattaagcaa caaatgtatt tgtgaagctt 4860
ggtttttagg ttgttgtgtt gtgtgtgctt gtgctctata ataatactat ccaggggctg 4920
gagaggtggc tcggagttca agagcacaga ctgctcttcc agaagtcctg agttcaattc 4980
ccagcaacca catggtggct cacaaccatc tgtaatggga tctgatgccc tcttctggtg 5040
tgtctgaaga ccacaagtgt attcacatta aataaataaa tcctccttct tcttcttttt 5100
ttttttttta aagagaatac tgtctccagt agaatttact gaagtaatga aatactttgt 5160
gtttgttcca atatggtagc caataatcaa attactcttt aagcactgga aatgttacca 5220
aggaactaat ttttatttga agtgtaactg tggacagagg agccataact gcagacttgt 5280
gggatacaga agaccaatgc agactttaat gtcttttctc ttacactaag caataaagaa 5340
ataaaaattg aacttctagt atcctatttg tttaaactgc tagctttact taacttttgt 5400
gcttcatcta tacaaagctg aaagctaagt ctgcagccat tactaaacat gaaagcaagt 5460
aatgataatt ttggatttca aaaatgtagg gccagagttt agccagccag tggtggtgct 5520
tgcctttatg cctttaatcc cagcactctg gaggcagaga caggcagatc tctgagtttg 5580
agcccagcct ggtctacaca tcaagttcta tctaggatag ccaggaatac acacagaaac 5640
cctgttgggg aggggggctc tgagatttca taaaattata attgaagcat tccctaatga 5700
gccactatgg atgtggctaa atccgtctac ctttctgatg agatttgggt attatttttt 5760
ctgtctctgc tgttggttgg gtcttttgac actgtgggct ttctttaaag cctccttcct 5820
gccatgtggt ctcttgtttg ctactaactt cccatggctt aaatggcatg gctttttgcc 5880
ttctaagggc agctgctgag atttgcagcc tgatttccag ggtggggttg ggaaatcttt 5940
caaacactaa aattgtcctt taattttttt tttaaaaaat gggttatata ataaacctca 6000
taaaatagtt atgaggagtg aggtggacta atattaaatg agtccctccc ctataaaaga 6060
gctattaagg ctttttgtct tatacttaac ttttttttta aatgtggtat ctttagaacc 6120
aagggtctta gagttttagt atacagaaac tgttgcatcg cttaatcaga ttttctagtt 6180
tcaaatccag agaatccaaa ttcttcacag ccaaagtcaa attaagaatt tctgactttt 6240
aatgttaatt tgcttactgt gaatataaaa atgatagctt ttcctgaggc agggtctcac 6300
tatgtatctc tgcctgatct gcaacaagat atgtagacta aagttctgcc tgcttttgtc 6360
tcctgaatac taaggttaaa atgtagtaat acttttggaa cttgcaggtc agattctttt 6420
ataggggaca cactaaggga gcttgggtga tagttggtaa aatgtgtttc aagtgatgaa 6480
aacttgaatt attatcaccg caacctactt tttaaaaaaa aaagccaggc ctgttagagc 6540
atgcttaagg gatccctagg acttgctgag cacacaagag tagttacttg gcaggctcct 6600
ggtgagagca tatttcaaaa aacaaggcag acaaccaaga aactacagtt aaggttacct 6660
gtctttaaac catctgcata tacacaggga tattaaaata ttccaaataa tatttcattc 6720
aagttttccc ccatcaaatt gggacatgga tttctccggt gaataggcag agttggaaac 6780
taaacaaatg ttggttttgt gatttgtgaa attgttttca agtgatagtt aaagcccatg 6840
agatacagaa caaagctgct atttcgaggt ctcttggttt atactcagaa gcacttcttt 6900
gggtttccct gcactatcct gatcatgtgc taggcctacc ttaggctgat tgttgttcaa 6960
ataaacttaa gtttcctgtc aggtgatgtc atatgatttc atatatcaag gcaaaacatg 7020
ttatatatgt taaacatttg tacttaatgt gaaagttagg tctttgtggg tttgattttt 7080
aattttcaaa acctgagcta aataagtcat ttttacatgt cttacatttg gtggaattgt 7140
ataattgtgg tttgcaggca agactctctg acctagtaac cctacctata gagcactttg 7200
ctgggtcaca agtctaggag tcaagcattt caccttgaag ttgagacgtt ttgttagtgt 7260
atactagttt atatgttgga ggacatgttt atccagaaga tattcaggac tatttttgac 7320
tgggctaagg aattgattct gattagcact gttagtgagc attgagtggc ctttaggctt 7380
gaattggagt cacttgtata tctcaaataa tgctggcctt ttttaaaaag cccttgttct 7440
ttatcaccct gttttctaca taatttttgt tcaaagaaat acttgtttgg atctcctttt 7500
gacaacaata gcatgttttc aagccatatt tttttccttt tttttttttt tttttggttt 7560
ttcgagacag ggtttctctg tatagccctg gctgtcctgg aactcacttt gtagaccagg 7620
ctggcctcga actcagaaat ccgcctgcct ctgcctcctg agtgccggga ttaaaggcgt 7680
gcaccaccac gcctggctaa gttggatatt ttgttatata actataacca atactaactc 7740
cactgggtgg atttttaatt cagtcagtag tcttaagtgg tctttattgg cccttcatta 7800
aaatctactg ttcactctaa cagaggctgt tggtactagt ggcacttaag caacttccta 7860
cggatatact agcagattaa gggtcaggga tagaaactag tctagcgttt tgtataccta 7920
ccagctttat actaccttgt tctgatagaa atatttcagg acatctagag tgtactataa 7980

ggttgatggt aagcttataa ggaacttgaa agtggagtaa ctactccatt tctctgaggg 8040
gagaattaaa atttttgacc aagtgttgtt gagccactga gaatggtctc agaacataac 8100
ttcttaagga accttcccag attgccctca acactgcacc acatttggtc ctgcttgaac 8160
attgccatgg ctcttaaagt cttaattaag aatattaatt gtgtaattat tgtttttcct 8220
cctttagatc attccttgag gacaggacag tgcttgttta aggctatatt tctgctgtct 8280
gagcagcaac aggtcttcga gatcaacatg atgttcataa tcccaagatg ttgccattta 8340
tgttctcaga agcaagcaga ggcatgatgg tcagtgacag taatgtcact gtgttaaatg 8400
ttgctatgca gtttggattt ttctaatgta gtgtaggtag aacatatgtg ttctgtatga 8460
attaaactct taagttacac cttgtataat ccatgcaatg tgttatgcaa ttaccatttt 8520
aagtattgta gctttctttg tatgtgagga taaaggtgtt tgtcataaaa tgttttgaac 8580
atttccccaa agttccaaat tataaaacca caacgttaga acttatttat gaacaatggt 8640
tgtagtttca tgcttttaaa atgcttaatt attcaattaa caccgtttgt gttataatat 8700
atataaaact gacatgtaga agtgtttgtc cagaacattt cttaaatgta tactgtcttt 8760
agagagttta atatagcatg tcttttgcaa catactaact tttgtgttgg tgcgagcaat 8820
attgtgtagt cattttgaaa ggagtcattt caatgagtgt cagattgttt tgaatgttat 8880
tgaacatttt aaatgcagac ttgttcgtgt tttagaaagc aaaactgtca gaagctttga 8940
actagaaatt aaaaagctga agtatttcag aagggaaata agctacttgc tgtattagtt 9000
gaaggaaagt gtaatagctt agaaaattta aaaccatata gttgtcattg ctgaatatct 9060
ggcagatgaa aagaaatact cagtggttct tttgagcaat ataacagctt gttatattaa 9120
aaattttccc cacagatata aactctaatc tataactcat aaatgttaca aatggatgaa 9180
gcttacaaat gtggcttgac ttgtcactgt gcttgtttta gttatgtgaa agtttggcaa 9240
taaacctatg tcctaaatag tcaaactgtg gaatgacttt ttaatctatt ggtttgtcta 9300
gaacagttat gttgccattt gccctaatgg tgaaagaaaa agtggggagt gccttggcac 9360
tgttcatttg tggtgtgaac caaagagggg ggcatgcact tacacttcaa acatcctttt 9420
gaaagactga caagtttggg tcttcacagt tggaattggg catccctttt gtcagggagg 9480
gagggaggga gggaggctgg cttgttatgc tgacaagtgt gattaaattc aaactttgag 9540
gtaagttgga ggaacttgta cattgttagg agtgtgacaa tttggactct taatgatttg 9600
gtcatacaaa atgaacctag accaacttct ggaagatgta tataataact ccatgttaca 9660
ttgatttcac ctgactaata cttatccctt atcaattaaa tacagaagat gccagccatc 9720
tgggcctttt aacccagaaa tttagtttca aactcctagg ttagtgttct cactgagcta 9780
catcctgatc tagtcctgaa aataggacca ccatcacccc caaaaaaatc tcaaataaga 9840
tttatgctag tgtttcaaaa ttttaggaat aggtaagatt agaaagtttt aaattttgag 9900
aaatggcttc tctagaaaga tgtacatagt gaacactgaa tggctcctaa agagcctaga 9960
aaactggtac tgagcacaca ggactgagag gtctttcttg aaaagcatgt attgctttac 10020
gtgggtcaca gaaggcaggc aggaagaact tgggctgaaa ctggtgtctt aagtggctaa 10080
catcttcaca actgatgagc aagaacttta tcctgatgca aaaaccatcc aaacaaacta 10140
agtgaaaggt ggcaatggat cccaggctgc tctagaggag gacttgactt ctcatcccat 10200
cacccacacc agatagctca tagactgcca attaacacca gcttctagcc tccacaggca 10260
cctgcactgg tacacataat ttcacacaaa cacagtaaga agccttccac ctggcatggt 10320
attgcttatc tttagttccc aacacttggg aggcagaggc cagccagggc tatgtgacaa 10380
aaaccttgtc tagaggagaa acttcatagc ttatttccta ttcacgtaac caggttagca 10440
aaatttacca gccagagatg aagctaacag tgtccactat atttgtagtg ttttaagtca 10500
attttttaaa tatacttaat agaattaaag ctatggtgaa ccaagtacaa acctggtgta 10560
ttaacttgag aacttagcat aaaaagtagt tcatttgttc agtaaatatt aaatgcttac 10620
tggcaaagat tatgtcagga acttggtaaa tggtgatgaa acaatcatag ttgtacatct 10680
tggttctgtg atcaccttgg tttgaggtaa aagtggttcc tttgatcaag gatggaattt 10740
taagtttata ttcaatcaat aatgtattat tttgtgattg caaaattgcc tatctagggt 10800
ataaaacctt taaaaatttc ataataccag ttcattctcc agttactaat tccaaaaagc 10860
cactgactat ggtgccaatg tggattctgt tctcaaagga aggattgtct gtgcccttta 10920
ttctaataga aacatcacac tgaaaatcta agctgaaaga agccagactt tcctaaataa 10980
ataactttcc ataaagctca aacaaggatt acttttagga ggcactgtta aggaactgat 11040
aagtaatgag gttacttata taatgatagt cccacaagac tatctgagga aaaatcagta 11100
caactcgaaa acagaacaac cagctaggca ggaataacag ggctcccaag tcaggaggtc 11160
tatccaacac ccttttctgt tgagggcccc agacctacat attgtataca aacagggagg 11220
tgggtgattt taactctcct gaggtacctt ggtaaatctt tgtcctgagt aagcagtaca 11280
gtgtacagtt tacattttca tttaaagata cattagctcc ctctaccccc taagactgac 11340
aggcactttg ggggtgggga gggctttgga aaataacgct tccatacact aaaagagaaa 11400
tttctttaat taggcttgtt ggttccatac atctactggt gtttctacta cttagtaata 11460
ttataatagt cacacaagca tctttgctct gtttaggttg tatatttatt ttaaggcaga 11520
tgataaaact gtagatctta agggatgctt ctgcttctga gatgatacaa agaatttaga 11580
ccataaaaca gtaggttgca caagcaatag aatatggcct aaagtgttct gacacttaga 11640

agccaagcag tgtaggcttc ttaagaaata ccattacaat caccttgcta gaaatcaagc 11700
attctggagt ggtcaagcag tgtaacctgt actgtaagtt acttttctgc tatttttctc 11760
ccaaagcaag ttctttatgc tgatatttcc agtgttagga actacaaata ttaataagtt 11820
gtcttcactc ttttctttac caaggagggt ctcttccttc atcttgatct gaaggatgaa 11880
caaaggcttg agcagtgcgc tttagaagat aaactgcagc atgaaggccc ccgatgttca 11940
cccagactac atggaccttt cgccacacat gtcccattcc agataaggcc tggcacacac 12000
aaaaaacata agtcattagg ctaccagtct gattctaaaa caacctaaaa tcttcccact 12060
taaatgctat gggtggtggg ttggaaagtt gactcagaaa atcacttgct gtttttagag 12120
aggatctggg ttcagtttct gatacattgt ggcttacaac tataactcca gttctagggg 12180
gtccatccaa catcctcttc tgttgagggc accaaataaa tgtattgtgt acaaacaggg 12240
aggtgagtga tttaactctc gtgtatagta ccttggtaaa acatttcttg tcctgagtaa 12300
gcagtacagc tctgcctgtc cctggtctac agacacggct catttcccga aggcaagctg 12360
gatagagatt ccaatttctc ttcttggatc ccatcctata aaagaaggtc aagtttaatc 12420
tattgcaaaa ggtaaatagg tagtttctta catgagacaa gaacaaatct taggtgtgaa 12480
gcagtcatct tttacaggcc agagcctcta ttctatgcca atgaaggaaa ctgttagtcc 12540
agtgttatag agttagtcca gtgtatagtt ttctatcaga acactttttt tttaaacaac 12600
tgcaacttag cttattgaag acaaaccacg agtagaaatc tgtccaagaa gcaagtgctt 12660
ctcagcctac aatgtggaat aggaccatgt aatggtacag tgagtgaaat gaattatggc 12720
atgtttttct gactgagaag acagtacaat aaaaggtaaa ctcatggtat ttatttaaaa 12780
agaatccaat ttctaccttt ttccaaatgg catatctgtt acaataatat ccacagaagc 12840
agttctcagt gggaggttgc agatatccca ctgaacagca tcaatgggca aaccccaggt 12900
tgtttttctg tggagacaaa ggtaagatat ttcaatatat tttcccaagc taatgagatg 12960
gctcagcaaa taatggtact ggccattaag tctcatgacc tgagcttgat cctcagggac 13020
catgtggtac aaggagagac ctaaatcctt cagttggact tcaatcttct accctcatgt 13080
ccacacacaa ataaatacaa taaaaaacat tctgcagtcg aatttctaaa agggcgaat 13139

<210> 4
<211> 1073
<212> DNA
<213> Mus musculus

<220>
<223> Description: 5' arm for Rosa26

<400> 4
caggccctcc gagcgtggtg gagccgttct gtgagacagc cgggtacgag tcgtgacgct 60
ggaaggggca agcgggtggt gggcaggaat gcggtccgcc ctgcagcaac cggaggggga 120
gggagaaggg agcggaaaag tctccaccgg acgcggccat ggctcggggg ggggggggca 180
gcggaggagc gcttccggcc gacgtctcgt cgctgattgg cttcttttcc tcccgccgtg 240
tgtgaaaaca caaatggcgt gttttggttg gcgtaaggcg cctgtcagtt aacggcagcc 300
ggagtgcgca gccgccggca gcctcgctct gcccactggg tggggcggga ggtaggtggg 360
gtgaggcgag ctggacgtgc gggcgcggtc ggcctctggc ggggcggggg aggggaggga 420
gggtcagcga aagtagctcg cgcgcgagcg gccgcccacc ctccccttcc tctgggggag 480
tcgttttacc cgccgccggc cgggcctcgt cgtctgattg gctctcgggg cccagaaaac 540
tggcccttgc cattggctcg tgttcgtgca agttgagtcc atccgccggc cagcgggggc 600
ggcgaggagg cgctcccagg ttccggccct cccctcggcc ccgcgccgca gagtctggcc 660
gcgcgcccct gcgcaacgtg gcaggaagcg cgcgctgggg gcggggacgg gcagtagggc 720
tgagcggctg cggggcgggt gcaagcacgt ttccgacttg agttgcctca agaggggcgt 780
gctgagccag acctccatcg cgcactccgg ggagtggagg gaaggagcga gggctcagtt 840
gggctgtttt ggaggcagga agcacttgct ctcccaaagt cgctctgagt tgttatcagt 900
aagggagctg cagtggagta ggcggggaga aggccgcacc cttctccgga ggggggaggg 960
gagtgttgca atacctttct gggagttctc tgctgcctcc tggcttctga ggaccgccct 1020
gggcctggga gaatcccttc cccctcttcc ctcgtgatct gcaactccag tct 1073

<210> 5
<211> 4333
<212> DNA
<213> Mus musculus

<220>
<223> Description: 3' arm for Rosa26

<400> 5
tagaagatgg gcgggagtct tctgggcagg cttaaaggct aacctggtgt gtgggcgttg 60
tcctgcaggg gaattgaaca ggtgtaaaat tggagggaca agacttccca cagattttcg 120
gttttgtcgg gaagtttttt aataggggca aataaggaaa atgggaggat aggtagtcat 180
ctggggtttt atgcagcaaa actacaggtt attattgctt gtgatccgcc tcggagtatt 240
ttccatcgag gtagattaaa gacatgctca cccgagtttt atactctcct gcttgagatc 300
cttactacag tatgaaatta cagtgtcgcg agttagacta tgtaagcaga attttaatca 360
tttttaaaga gcccagtact tcatatccat ttctcccgct ccttctgcag ccttatcaaa 420
aggtatttta gaacactcat tttagcccca ttttcattta ttatactggc ttatccaacc 480
cctagacaga gcattggcat tttccctttc ctgatcttag aagtctgatg actcatgaaa 540
ccagacagat tagttacata caccacaaat cgaggctgta gctggggcct caacactgca 600
gttcttttat aactccttag tacacttttt gttgatcctt tgccttgatc cttaattttc 660
agtgtctatc acctctcccg tcagtggtgt tccacatttg ggcctattct cagtccaggg 720
agttttacaa caatagatgt attgagaatc caacctaaag cttaactttc cactcccatg 780
aatgcctctc tcctttttct ccatttataa actgagctat taaccattaa tggttccagg 840
tggatgtctc ctccccatat tacctgatgt atcttacata ttgccaggct gatattttaa 900
gacattaaaa ggtatatttc attattgagc cacatggtat tgattactgc ttactaaaat 960
tttgtcattg tacacatctg taaaaggtgg ttccttttgg aatgcaaagt tcaggtgttt 1020
gttgtctttc ctgacctaag gtcttgtgag cttgtatttt ttctatttaa gcagtgcttt 1080
ctcttggact ggcttgactc atggcattct acacgttatt gctggtctaa atgtgatttt 1140
gccaagcttc ttcaggacct ataattttgc ttgacttgta gccaaacaca agtaaaatga 1200
ttaagcaaca aatgtatttg tgaagcttgg tttttaggtt gttgtgttgt gtgtgcttgt 1260
gctctataat aatactatcc aggggctgga gaggtggctc ggagttcaag agcacagact 1320
gctcttccag aagtcctgag ttcaattccc agcaaccaca tggtggctca caaccatctg 1380
taatgggatc tgatgccctc ttctggtgtg tctgaagacc acaagtgtat tcacattaaa 1440
taaataaatc ctccttcttc ttcttttttt tttttttaaa gagaatactg tctccagtag 1500
aatttactga agtaatgaaa tactttgtgt ttgttccaat atggtagcca ataatcaaat 1560
tactctttaa gcactggaaa tgttaccaag gaactaattt ttatttgaag tgtaactgtg 1620
gacagaggag ccataactgc agacttgtgg gatacagaag accaatgcag actttaatgt 1680
cttttctctt acactaagca ataaagaaat aaaaattgaa cttctagtat cctatttgtt 1740
taaactgcta gctttactta acttttgtgc ttcatctata caaagctgaa agctaagtct 1800
gcagccatta ctaaacatga aagcaagtaa tgataatttt ggatttcaaa aatgtagggc 1860
cagagtttag ccagccagtg gtggtgcttg cctttatgcc tttaatccca gcactctgga 1920
ggcagagaca ggcagatctc tgagtttgag cccagcctgg tctacacatc aagttctatc 1980
taggatagcc aggaatacac acagaaaccc tgttggggag gggggctctg agatttcata 2040
aaattataat tgaagcattc cctaatgagc cactatggat gtggctaaat ccgtctacct 2100
ttctgatgag atttgggtat tattttttct gtctctgctg ttggttgggt cttttgacac 2160
tgtgggcttt ctttaaagcc tccttcctgc catgtggtct cttgtttgct actaacttcc 2220
catggcttaa atggcatggc tttttgcctt ctaagggcag ctgctgagat ttgcagcctg 2280
atttccaggg tggggttggg aaatctttca aacactaaaa ttgtccttta attttttttt 2340
taaaaaatgg gttatataat aaacctcata aaatagttat gaggagtgag gtggactaat 2400
attaaatgag tccctcccct ataaaagagc tattaaggct ttttgtctta tacttaactt 2460
tttttttaaa tgtggtatct ttagaaccaa gggtcttaga gttttagtat acagaaactg 2520
ttgcatcgct taatcagatt ttctagtttc aaatccagag aatccaaatt cttcacagcc 2580
aaagtcaaat taagaatttc tgacttttaa tgttaatttg cttactgtga atataaaaat 2640
gatagctttt cctgaggcag ggtctcacta tgtatctctg cctgatctgc aacaagatat 2700
gtagactaaa gttctgcctg cttttgtctc ctgaatacta aggttaaaat gtagtaatac 2760
ttttggaact tgcaggtcag attcttttat aggggacaca ctaagggagc ttgggtgata 2820
gttggtaaaa tgtgtttcaa gtgatgaaaa cttgaattat tatcaccgca acctactttt 2880
taaaaaaaaa agccaggcct gttagagcat gcttaaggga tccctaggac ttgctgagca 2940
cacaagagta gttacttggc aggctcctgg tgagagcata tttcaaaaaa caaggcagac 3000
aaccaagaaa ctacagttaa ggttacctgt ctttaaacca tctgcatata cacagggata 3060
ttaaaatatt ccaaataata tttcattcaa gttttccccc atcaaattgg gacatggatt 3120
tctccggtga ataggcagag ttggaaacta aacaaatgtt ggttttgtga tttgtgaaat 3180
tgttttcaag tgatagttaa agcccatgag atacagaaca aagctgctat ttcgaggtct 3240
cttggtttat actcagaagc acttctttgg gtttccctgc actatcctga tcatgtgcta 3300
ggcctacctt aggctgattg ttgttcaaat aaacttaagt ttcctgtcag gtgatgtcat 3360
atgatttcat atatcaaggc aaaacatgtt atatatgtta aacatttgta cttaatgtga 3420

aagttaggtc tttgtgggtt tgatttttaa ttttcaaaac ctgagctaaa taagtcattt 3480
ttacatgtct tacatttggt ggaattgtat aattgtggtt tgcaggcaag actctctgac 3540
ctagtaaccc tacctataga gcactttgct gggtcacaag tctaggagtc aagcatttca 3600
ccttgaagtt gagacgtttt gttagtgtat actagtttat atgttggagg acatgtttat 3660
ccagaagata ttcaggacta tttttgactg ggctaaggaa ttgattctga ttagcactgt 3720
tagtgagcat tgagtggcct ttaggcttga attggagtca cttgtatatc tcaaataatg 3780
ctggcctttt ttaaaaagcc cttgttcttt atcaccctgt tttctacata atttttgttc 3840
aaagaaatac ttgtttggat ctccttttga caacaatagc atgttttcaa gccatatttt 3900
ttttcctttt tttttttttt tttggttttt cgagacaggg tttctctgta tagccctggc 3960
tgtcctggaa ctcactttgt agaccaggct ggcctcgaac tcagaaatcc gcctgcctct 4020
gcctcctgag tgccgggatt aaaggcgtgc accaccacgc ctggctaagt tggatatttt 4080
gttatataac tataaccaat actaactcca ctgggtggat ttttaattca gtcagtagtc 4140
ttaagtggtc tttattggcc cttcattaaa atctactgtt cactctaaca gaggctgttg 4200
gtactagtgg cacttaagca acttcctacg gatatactag cagattaagg gtcagggata 4260
gaaactagtc tagcgttttg tatacctacc agctttatac taccttgttc tgatagaaat 4320
atttcaggac atc 4333

<210> 6
<211> 6039
<212> DNA
<213> Artificial Sequence

<220>
<223> Description of Artificial Sequence: Insert
      CAGGAS-creER

<400> 6
attgattatt gactagttat taatagtaat caattacggg gtcattagtt catagcccat 60
atatggagtt ccgcgttaca taacttacgg taaatggccc gcctggctga ccgcccaacg 120
acccccgccc attgacgtca ataatgacgt atgttcccat agtaacgcca atagggactt 180
tccattgacg tcaatgggtg gactatttac ggtaaactgc ccacttggca gtacatcaag 240
tgtatcatat gccaagtacg ccccctattg acgtcaatga cggtaaatgg cccgcctggc 300
attatgccca gtacatgacc ttatgggact ttcctacttg gcagtacatc tacgtattag 360
tcatcgctat taccatgggt cgaggtgagc cccacgttct gcttcactct ccccatctcc 420
cccccctccc cacccccaat tttgtattta tttatttttt aattattttg tgcagcgatg 480
ggggcggggg gggggggggc gcgcgccagg cggggcgggg cggggcgagg ggcggggcgg 540
ggcgaggcgg agaggtgcgg cggcagccaa tcagagcggc gcgctccgaa agtttccttt 600
tatggcgagg cggcggcggc ggcggcccta taaaaagcga agcgcgcggc gggcgggagt 660
cgctgcgttg ccttcgcccc gtgccccgct ccgcgccgcc tcgcgccgcc cgccccggct 720
ctgactgacc gcgttactcc cacaggtgag cgggcgggac ggcccttctc ctccgggctg 780
taattagcgc ttggtttaat gacggctcgt ttcttttctg tggctgcgtg aaagccttaa 840
agggctccgg gagggccctt tgtgcggggg ggagcggctc ggggggtgcg tgcgtgtgtg 900
tgtgcgtggg gagcgccgcg tgcggcccgc gctgcccggc ggctgtgagc gctgcgggcg 960
cggcgcgggg ctttgtgcgc tccgcgtgtg cgcgagggga gcgcggccgg gggcggtgcc 1020
ccgcggtgcg ggggggctgc gaggggaaca aaggctgcgt gcggggtgtg tgcgtggggg 1080
ggtgagcagg gggtgtgggc gcggcggtcg ggctgtaacc cccccctgca cccccctccc 1140
cgagttgctg agcacggccc ggcttcgggt gcggggctcc gtgcggggcg tggcgcgggg 1200
ctcgccgtgc cgggcggggg gtggcggcag gtgggggtgc cgggcggggc ggggccgcct 1260
cgggccgggg agggctcggg ggaggggcgc ggcggccccg gagcgccggc ggctgtcgag 1320
gcgcggcgag ccgcagccat tgccttttat ggtaatcgtg cgagagggcg cagggacttc 1380
ctttgtccca aatctggcgg agccgaaatc tgggaggcgc cgccgcaccc cctctagcgg 1440
gcgcgggcga agcggtgcgg cgccggcagg aaggaaatgg gcggggaggg ccttcgtgcg 1500
tcgccgcgcc gccgtcccct tctccatctc cagcctcggg gctgccgcag ggggacggct 1560
gccttcgggg gggacggggc agggcggggt tcggcttctg gcgtgtgacc ggcggctcta 1620
gaagcgttgg ggtgagtact ccctctcaaa agcgggcatg acttctgcgc taagattgtc 1680
agtttccaaa aacgaggagg atttgatatt cacctggccc gcggtgatgc ctttgagggt 1740
ggccgcgtcc atctggtcag aaaagacaat ctttttgttg tcaagcttga ggtgtggcag 1800
gcttgagatc tggccataca cttgagtgac attgacatcc actttgcctt tctctccaca 1860
ggtgtccact cccagggcgg cctccggagc gatcgccggt ccgcctaggc tagccggccg 1920
cgtcgaccat gtccaattta ctgaccgtac accaaaattt gcctgcatta ccggtcgatg 1980

caacgagtga tgaggttcgc aagaacctga tggacatgtt cagggatcgc caggcgtttt 2040
ctgagcatac ctggaaaatg cttctgtccg tttgccggtc gtgggcggca tggtgcaagt 2100
tgaataaccg gaaatggttt cccgcagaac ctgaagatgt tcgcgattat cttctatatc 2160
ttcaggcgcg cggtctggca gtaaaaacta tccagcaaca tttgggccag ctaaacatgc 2220
ttcatcgtcg gtccgggctg ccacgaccaa gtgacagcaa tgctgtttca ctggttatgc 2280
ggcggatccg aaaagaaaac gttgatgccg gtgaacgtgc aaaacaggct ctagcgttcg 2340
aacgcactga tttcgaccag gttcgttcac tcatggaaaa tagcgatcgc tgccaggata 2400
tacgtaatct ggcatttctg gggattgctt ataacaccct gttacgtata gccgaaattg 2460
ccaggatcag ggttaaagat atctcacgta ctgacggtgg gagaatgtta atccatattg 2520
gcagaacgaa aacgctggtt agcaccgcag gtgtagagaa ggcacttagc ctgggggtaa 2580
ctaaactggt cgagcgatgg atttccgtct ctggtgtagc tgatgatccg aataactacc 2640
tgttttgccg ggtcagaaaa aatggtgttg ccgcgccatc tgccaccagc cagctatcaa 2700
ctcgcgccct ggaagggatt tttgaagcaa ctcatcgatt gatttacggc gctaaggatg 2760
actctggtca gagatacctg gcctggtctg gacacagtgc ccgtgtcgga gccgcgcgag 2820
atatggcccg cgctggagtt tcaataccgg agatcatgca agctggtggc tggaccaatg 2880
taaatattgt catgaactat atccgtaacc tggatagtga aacaggggca atggtgcgcc 2940
tgctggaaga tggcgattct gctggagaca tgagagctgc caacctttgg ccaagcccgc 3000
tcatgatcaa acgctctaag aagaacagcc tggccttgtc cctgacggcc gaccagatgg 3060
tcagtgcctt gttggatgct gagcccccca tactctattc cgagtatgat cctaccagac 3120
ccttcagtga agcttcgatg atgggcttac tgaccaacct ggcagacagg gagctggttc 3180
acatgatcaa ctgggcgaag agggtgccag gctttgtgga tttgaccctc catgatcagg 3240
tccaccttct agaatgtgcc tggctagaga tcctgatgat tggtctcgtc tggcgctcca 3300
tggagcaccc agtgaagcta ctgtttgctc ctaacttgct cttggacagg aaccagggaa 3360
aatgtgtaga gggcatggtg gagatcttcg acatgctgct ggctacatca tctcggttcc 3420
gcatgatgaa tctgcaggga gaggagtttg tgtgcctcaa atctattatt ttgcttaatt 3480
ctggagtgta cacatttctg tccagcaccc tgaagtctct ggaagagaag gaccatatcc 3540
accgagtcct ggacaagatc acagacactt tgatccacct gatggccaag gcaggcctga 3600
ccctgcagca gcagcaccag cggctggccc agctcctcct catcctctcc cacatcaggc 3660
acatgagtaa caaaggcatg gagcatctgt acagcatgaa gtgcaagaac gtggtgcccc 3720
tctatgacct gctgctggag gcggccgacg cccaccgcct acatgcgccc actagccgtg 3780
gaggggcatc cgtggaggag acggaccaaa gccacttggc cactgcgggc tctacttcat 3840
cgcattcctt gcaaaagtat tacatcacgg gggaggcaga gggtttccct gccacagtct 3900
gagcggccga ccggttcgag atccaggcgc ggatcaataa aagatcatta ttttcaatag 3960
atctgtgtgt tggttttttg tgtgccttgg gggaggggga ggccagaatg aggcgcggcc 4020
aagggggagg gggaggccag aatgaccttg gggagggggg aggccagaat gaccttgggg 4080
gagggggagg ccagaatgag gcgcgccggt aaccgaagtt cctatacttt ctagagaata 4140
ggaacttcgg aataggaact tcttaggtca attctaccgg gtaggggagg cgcttttccc 4200
aaggcagtct ggagcatgcg ctttagcagc cccgctgggc acttggcgct acacaagtgg 4260
cctctggcct cgcacacatt ccacatccac cggtaggcgc caaccggctc cgttctttgg 4320
tggccccttc gcgccacctt ctactcctcc cctagtcagg aagttccccc ccgccccgca 4380
gctcgcgtcg tgcaggacgt gacaaatgga agtagcacgt ctcactagtc tcgtgcagat 4440
ggacagcacc gctgagcaat ggaagcgggt aggcctttgg ggcagcggcc aatagcagct 4500
ttgctccttc gctttctggg ctcagaggct gggaaggggt gggtccgggg gcgggctcag 4560
gggcgggctc aggggcgggg cgggcgcccg aaggtcctcc ggaggcccgg cattctgcac 4620
gcttcaaaag cgcacgtctg ccgcgctgtt ctcctcttcc tcatctccgg gcctttcgac 4680
ctgcagccaa tatgggatcg gccattgaac aagatggatt gcacgcaggt tctccggccg 4740
cttgggtgga gaggctattc ggctatgact gggcacaaca gacaatcggc tgctctgatg 4800
ccgccgtgtt ccggctgtca gcgcaggggc gcccggttct ttttgtcaag accgacctgt 4860
ccggtgccct gaatgaactg caggacgagg cagcgcggct atcgtggctg gccacgacgg 4920
gcgttccttg cgcagctgtg ctcgacgttg tcactgaagc gggaagggac tggctgctat 4980
tgggcgaagt gccggggcag gatctcctgt catctcacct tgctcctgcc gagaaagtat 5040
ccatcatggc tgatgcaatg cggcggctgc atacgcttga tccggctacc tgcccattcg 5100
accaccaagc gaaacatcgc atcgagcgag cacgtactcg gatggaagcc ggtcttgtcg 5160
atcaggatga tctggacgaa gagcatcagg ggctcgcgcc agccgaactg ttcgccaggc 5220
tcaaggcgcg catgcccgac ggcgaggatc tcgtcgtgac ccatggcgat gcctgcttgc 5280
cgaatatcat ggtggaaaat ggccgctttt ctggattcat cgactgtggc cggctgggtg 5340
tggcggaccg ctatcaggac atagcgttgg ctacccgtga tattgctgaa gagcttggcg 5400
gcgaatgggc tgaccgcttc ctcgtgcttt acggtatcgc cgctcccgat tcgcagcgca 5460
tcgccttcta tcgccttctt gacgagttct tctgagggga tcgatccgct gtaagtctgc 5520
agaaattgat gatctattaa acaataaaga tgtccactaa aatggaagtt tttcctgtca 5580
tactttgtta agaagggtga gaacagagta cctacatttt gaatggaagg attggagcta 5640

cgggggtggg ggtggggtgg gattagataa atgcctgctc tttactgaag gctctttact 5700
attgctttat gataatgttt catagttgga tatcataatt taaacaagca aaaccaaatt 5760
aagggccagc tcattcctcc cactcatgat ctatagatct atagatctct cgtgggatca 5820
ttgtttttct cttgattccc actttgtggt tctaagtact gtggtttcca aatgtgtcag 5880
tttcatagcc tgaagaacga gatcagcagc ctctgttcca catacacttc attctcagta 5940
ttgttttgcc aagttctaat tccatcagaa gctgactcta gatcccgcgc cgaagttcct 6000
atactttcta gagaatagga acttcggaat aggaacttc 6039

<210> 7
<211> 14411
<212> DNA
<213> Artificial Sequence

<220>
<223> Description of Artificial Sequence: targeting
      vector for Rosa26 locus with a CAGGS-creER insert

<400> 7
tttgagtgag ctgataccgc tcgccgcagc cgaacgaccg agcgcagcga gtcagtgagc 60
gaggaagcgg aagagcgccc aatacgcaaa ccgcctctcc ccgcgcgttg gccgattcat 120
taatgcagct ggcacgacag gtttcccgac tggaaagcgg gcagtgagcg caacgcaatt 180
aatgtgagtt agctcactca ttaggcaccc caggctttac actttatgct tccggctcgt 240
atgttgtgtg gaattgtgag cggataacaa tttcacacag gaaacagcta tgaccatgat 300
tacgccaagc gcgcaattaa ccctcactaa agggaacaaa agctgtcgag atctagatat 360
cgatggccat agagttacgc tagggataac agggtaatat agccgcggca ggccctccga 420
gcgtggtgga gccgttctgt gagacagccg ggtacgagtc gtgacgctgg aaggggcaag 480
cgggtggtgg gcaggaatgc ggtccgccct gcagcaaccg gagggggagg gagaagggag 540
cggaaaagtc tccaccggac gcggccatgg ctcggggggg ggggggcagc ggaggagcgc 600
ttccggccga cgtctcgtcg ctgattggct tcttttcctc ccgccgtgtg tgaaaacaca 660
aatggcgtgt tttggttggc gtaaggcgcc tgtcagttaa cggcagccgg agtgcgcagc 720
cgccggcagc ctcgctctgc ccactgggtg gggcgggagg taggtggggt gaggcgagct 780
ggacgtgcgg gcgcggtcgg cctctggcgg ggcgggggag ggagggaggg gtcagcgaaa 840
gtagctcgcg cgcgagcggc cgcccaccct ccccttcctc tgggggagtc gttttacccg 900
ccgccggccg ggcctcgtcg tctgattggc tctcggggcc cagaaaactg gcccttgcca 960
ttggctcgtg ttcgtgcaag ttgagtccat ccgccggcca gcgggggcgg cgaggaggcg 1020
ctcccaggtt ccggccctcc cctcggcccc gcgccgcaga gtctggccgc gcgcccctgc 1080
gcaacgtggc aggaagcgcg cgctgggggc ggggacgggc agtagggctg agcggctgcg 1140
gggcgggtgc aagcacgttt ccgacttgag ttgcctcaag aggggcgtgc tgagccagac 1200
ctccatcgcg cactccgggg agtggaggga aggagcgagg gctcagttgg gctgttttgg 1260
aggcaggaag cacttgctct cccaaagtcg ctctgagttg ttatcagtaa gggagctgca 1320
gtggagtagg cggggagaag gccgcaccct tctccggagg ggggagggga gtgttgcaat 1380
acctttctgg gagttctctg ctgcctcctg gcttctgagg accgccctgg gcctgggaga 1440
atcccttccc cctcttccct cgtgatctgc aactccagtc tttctaggta accgatatcc 1500
ctgcaggttt tcgacattga ttattgacta gttattaata gtaatcaatt acggggtcat 1560
tagttcatag cccatatatg gagttccgcg ttacataact tacggtaaat ggcccgcctg 1620
gctgaccgcc caacgacccc cgcccattga cgtcaataat gacgtatgtt cccatagtaa 1680
cgccaatagg gactttccat tgacgtcaat gggtggacta tttacggtaa actgcccact 1740
tggcagtaca tcaagtgtat catatgccaa gtacgccccc tattgacgtc aatgacggta 1800
aatggcccgc ctggcattat gcccagtaca tgaccttatg ggactttcct acttggcagt 1860
acatctacgt attagtcatc gctattacca tgggtcgagg tgagccccac gttctgcttc 1920
actctcccca tctccccccc ctccccaccc ccaattttgt atttatttat tttttaatta 1980
ttttgtgcag cgatgggggc gggggggggg ggggcgcgcg ccaggcgggg cggggcgggg 2040
cgaggggcgg ggcggggcga ggcggagagg tgcggcggca gccaatcaga gcggcgcgct 2100
ccgaaagttt ccttttatgg cgaggcggcg gcggcggcgg ccctataaaa agcgaagcgc 2160
gcggcgggcg ggagtcgctg cgttgccttc gccccgtgcc ccgctccgcg ccgcctcgcg 2220
ccgcccgccc cggctctgac tgaccgcgtt actcccacag gtgagcgggc gggacggccc 2280
ttctcctccg ggctgtaatt agcgcttggt ttaatgacgg ctcgtttctt ttctgtggct 2340
gcgtgaaagc cttaaagggc tccgggaggg ccctttgtgc gggggggagc ggctcggggg 2400
gtgcgtgcgt gtgtgtgtgc gtggggagcg ccgcgtgcgg cccgcgctgc ccggcggctg 2460
tgagcgctgc gggcgcggcg cggggctttg tgcgctccgc gtgtgcgcga ggggagcgcg 2520

gccgggggcg gtgccccgcg gtgcgggggg gctgcgaggg gaacaaaggc tgcgtgcggg 2580
gtgtgtgcgt gggggggtga gcagggggtg tgggcgcggc ggtcgggctg taaccccccc 2640
ctgcaccccc ctccccgagt tgctgagcac ggcccggctt cgggtgcggg gctccgtgcg 2700
gggcgtggcg cggggctcgc cgtgccgggc ggggggtggc ggcaggtggg ggtgccgggc 2760
ggggcggggc cgcctcgggc cggggagggc tcgggggagg ggcgcggcgg ccccggagcg 2820
ccggcggctg tcgaggcgcg gcgagccgca gccattgcct tttatggtaa tcgtgcgaga 2880
gggcgcaggg acttcctttg tcccaaatct ggcggagccg aaatctggga ggcgccgccg 2940
caccccctct agcgggcgcg ggcgaagcgg tgcggcgccg gcaggaagga aatgggcggg 3000
gagggccttc gtgcgtcgcc gcgccgccgt ccccttctcc atctccagcc tcggggctgc 3060
cgcaggggga cggctgcctt cgggggggac ggggcagggc ggggttcggc ttctggcgtg 3120
tgaccggcgg ctctagaagc gttggggtga gtactccctc tcaaaagcgg gcatgacttc 3180
tgcgctaaga ttgtcagttt ccaaaaacga ggaggatttg atattcacct ggcccgcggt 3240
gatgcctttg agggtggccg cgtccatctg gtcagaaaag acaatctttt tgttgtcaag 3300
cttgaggtgt ggcaggcttg agatctggcc atacacttga gtgacattga catccacttt 3360
gcctttctct ccacaggtgt ccactcccag ggcggcctcc ggagcgatcg ccggtccgcc 3420
taggctagcc ggccgcgtcg accatgtcca atttactgac cgtacaccaa aatttgcctg 3480
cattaccggt cgatgcaacg agtgatgagg ttcgcaagaa cctgatggac atgttcaggg 3540
atcgccaggc gttttctgag catacctgga aaatgcttct gtccgtttgc cggtcgtggg 3600
cggcatggtg caagttgaat aaccggaaat ggtttcccgc agaacctgaa gatgttcgcg 3660
attatcttct atatcttcag gcgcgcggtc tggcagtaaa aactatccag caacatttgg 3720
gccagctaaa catgcttcat cgtcggtccg ggctgccacg accaagtgac agcaatgctg 3780
tttcactggt tatgcggcgg atccgaaaag aaaacgttga tgccggtgaa cgtgcaaaac 3840
aggctctagc gttcgaacgc actgatttcg accaggttcg ttcactcatg gaaaatagcg 3900
atcgctgcca ggatatacgt aatctggcat ttctggggat tgcttataac accctgttac 3960
gtatagccga aattgccagg atcagggtta aagatatctc acgtactgac ggtgggagaa 4020
tgttaatcca tattggcaga acgaaaacgc tggttagcac cgcaggtgta gagaaggcac 4080
ttagcctggg ggtaactaaa ctggtcgagc gatggatttc cgtctctggt gtagctgatg 4140
atccgaataa ctacctgttt tgccgggtca gaaaaaatgg tgttgccgcg ccatctgcca 4200
ccagccagct atcaactcgc gccctggaag ggatttttga agcaactcat cgattgattt 4260
acggcgctaa ggatgactct ggtcagagat acctggcctg gtctggacac agtgcccgtg 4320
tcggagccgc gcgagatatg gcccgcgctg gagtttcaat accggagatc atgcaagctg 4380
gtggctggac caatgtaaat attgtcatga actatatccg taacctggat agtgaaacag 4440
gggcaatggt gcgcctgctg gaagatggcg attctgctgg agacatgaga gctgccaacc 4500
tttggccaag cccgctcatg atcaaacgct ctaagaagaa cagcctggcc ttgtccctga 4560
cggccgacca gatggtcagt gccttgttgg atgctgagcc ccccatactc tattccgagt 4620
atgatcctac cagacccttc agtgaagctt cgatgatggg cttactgacc aacctggcag 4680
acagggagct ggttcacatg atcaactggg cgaagagggt gccaggcttt gtggatttga 4740
ccctccatga tcaggtccac cttctagaat gtgcctggct agagatcctg atgattggtc 4800
tcgtctggcg ctccatggag cacccagtga agctactgtt tgctcctaac ttgctcttgg 4860
acaggaacca gggaaaatgt gtagagggca tggtggagat cttcgacatg ctgctggcta 4920
catcatctcg gttccgcatg atgaatctgc agggagagga gtttgtgtgc ctcaaatcta 4980
ttattttgct taattctgga gtgtacacat ttctgtccag caccctgaag tctctggaag 5040
agaaggacca tatccaccga gtcctggaca agatcacaga cactttgatc cacctgatgg 5100
ccaaggcagg cctgaccctg cagcagcagc accagcggct ggcccagctc ctcctcatcc 5160
tctcccacat caggcacatg agtaacaaag gcatggagca tctgtacagc atgaagtgca 5220
agaacgtggt gcccctctat gacctgctgc tggaggcggc cgacgcccac cgcctacatg 5280
cgcccactag ccgtggaggg gcatccgtgg aggagacgga ccaaagccac ttggccactg 5340
cgggctctac ttcatcgcat tccttgcaaa agtattacat cacgggggag gcagagggtt 5400
tccctgccac agtctgagcg gccgaccggt tcgagatcca ggcgcggatc aataaaagat 5460
cattattttc aatagatctg tgtgttggtt ttttgtgtgc cttgggggag ggggaggcca 5520
gaatgaggcg cggccaaggg ggagggggag gccagaatga ccttggggga gggggaggcc 5580
agaatgacct tgggggaggg ggaggccaga atgaggcgcg ccggtaaccg aagttcctat 5640
actttctaga gaataggaac ttcggaatag gaacttctta ggtcaattct accgggtagg 5700
ggaggcgctt ttcccaaggc agtctggagc atgcgcttta gcagccccgc tgggcacttg 5760
gcgctacaca agtggcctct ggcctcgcac acattccaca tccaccggta ggcgccaacc 5820
ggctccgttc tttggtggcc ccttcgcgcc accttctact cctcccctag tcaggaagtt 5880
cccccccgcc ccgcagctcg cgtcgtgcag gacgtgacaa atggaagtag cacgtctcac 5940
tagtctcgtg cagatggaca gcaccgctga gcaatggaag cgggtaggcc tttggggcag 6000
cggccaatag cagctttgct ccttcgcttt ctgggctcag aggctgggaa ggggtgggtc 6060
cgggggcggg ctcaggggcg ggctcagggg cggggcgggc gcccgaaggt cctccggagg 6120
cccggcattc tgcacgcttc aaaagcgcac gtctgccgcg ctgttctcct cttcctcatc 6180

tccgggcctt tcgacctgca gccaatatgg gatcggccat tgaacaagat ggattgcacg 6240
caggttctcc ggccgcttgg gtggagaggc tattcggcta tgactgggca caacagacaa 6300
tcggctgctc tgatgccgcc gtgttccggc tgtcagcgca ggggcgcccg gttctttttg 6360
tcaagaccga cctgtccggt gccctgaatg aactgcagga cgaggcagcg cggctatcgt 6420
ggctggccac gacgggcgtt ccttgcgcag ctgtgctcga cgttgtcact gaagcgggaa 6480
gggactggct gctattgggc gaagtgccgg ggcaggatct cctgtcatct caccttgctc 6540
ctgccgagaa agtatccatc atggctgatg caatgcggcg gctgcatacg cttgatccgg 6600
ctacctgccc attcgaccac caagcgaaac atcgcatcga gcgagcacgt actcggatgg 6660
aagccggtct tgtcgatcag gatgatctgg acgaagagca tcaggggctc gcgccagccg 6720
aactgttcgc caggctcaag gcgcgcatgc ccgacggcga ggatctcgtc gtgacccatg 6780
gcgatgcctg cttgccgaat atcatggtgg aaaatggccg cttttctgga ttcatcgact 6840
gtggccggct gggtgtggcg gaccgctatc aggacatagc gttggctacc cgtgatattg 6900
ctgaagagct tggcggcgaa tgggctgacc gcttcctcgt gctttacggt atcgccgctc 6960
ccgattcgca gcgcatcgcc ttctatcgcc ttcttgacga gttcttctga ggggatcgat 7020
ccgctgtaag tctgcagaaa ttgatgatct attaaacaat aaagatgtcc actaaaatgg 7080
aagtttttcc tgtcatactt tgttaagaag ggtgagaaca gagtacctac attttgaatg 7140
gaaggattgg agctacgggg gtgggggtgg ggtgggatta gataaatgcc tgctctttac 7200
tgaaggctct ttactattgc tttatgataa tgtttcatag ttggatatca taatttaaac 7260
aagcaaaacc aaattaaggg ccagctcatt cctcccactc atgatctata gatctataga 7320
tctctcgtgg gatcattgtt tttctcttga ttcccacttt gtggttctaa gtactgtggt 7380
ttccaaatgt gtcagtttca tagcctgaag aacgagatca gcagcctctg ttccacatac 7440
acttcattct cagtattgtt ttgccaagtt ctaattccat cagaagctga ctctagatcc 7500
cgcgccgaag ttcctatact ttctagagaa taggaacttc ggaataggaa cttcaagctt 7560
aagcgctaga agatgggcgg gagtcttctg ggcaggctta aaggctaacc tggtgtgtgg 7620
gcgttgtcct gcaggggaat tgaacaggtg taaaattgga gggacaagac ttcccacaga 7680
ttttcggttt tgtcgggaag ttttttaata ggggcaaata aggaaaatgg gaggataggt 7740
agtcatctgg ggttttatgc agcaaaacta caggttatta ttgcttgtga tccgcctcgg 7800
agtattttcc atcgaggtag attaaagaca tgctcacccg agttttatac tctcctgctt 7860
gagatcctta ctacagtatg aaattacagt gtcgcgagtt agactatgta agcagaattt 7920
taatcatttt taaagagccc agtacttcat atccatttct cccgctcctt ctgcagcctt 7980
atcaaaaggt attttagaac actcatttta gccccatttt catttattat actggcttat 8040
ccaaccccta gacagagcat tggcattttc cctttcctga tcttagaagt ctgatgactc 8100
atgaaaccag acagattagt tacatacacc acaaatcgag gctgtagctg gggcctcaac 8160
actgcagttc ttttataact ccttagtaca ctttttgttg atcctttgcc ttgatcctta 8220
attttcagtg tctatcacct ctcccgtcag tggtgttcca catttgggcc tattctcagt 8280
ccagggagtt ttacaacaat agatgtattg agaatccaac ctaaagctta actttccact 8340
cccatgaatg cctctctcct ttttctccat ttataaactg agctattaac cattaatggt 8400
tccaggtgga tgtctcctcc ccatattacc tgatgtatct tacatattgc caggctgata 8460
ttttaagaca ttaaaaggta tatttcatta ttgagccaca tggtattgat tactgcttac 8520
taaaattttg tcattgtaca catctgtaaa aggtggttcc ttttggaatg caaagttcag 8580
gtgtttgttg tctttcctga cctaaggtct tgtgagcttg tattttttct atttaagcag 8640
tgctttctct tggactggct tgactcatgg cattctacac gttattgctg gtctaaatgt 8700
gattttgcca agcttcttca ggacctataa ttttgcttga cttgtagcca aacacaagta 8760
aaatgattaa gcaacaaatg tatttgtgaa gcttggtttt taggttgttg tgttgtgtgt 8820
gcttgtgctc tataataata ctatccaggg gctggagagg tggctcggag ttcaagagca 8880
cagactgctc ttccagaagt cctgagttca attcccagca accacatggt ggctcacaac 8940
catctgtaat gggatctgat gccctcttct ggtgtgtctg aagaccacaa gtgtattcac 9000
attaaataaa taaatcctcc ttcttcttct tttttttttt tttaaagaga atactgtctc 9060
cagtagaatt tactgaagta atgaaatact ttgtgtttgt tccaatatgg tagccaataa 9120
tcaaattact ctttaagcac tggaaatgtt accaaggaac taatttttat ttgaagtgta 9180
actgtggaca gaggagccat aactgcagac ttgtgggata cagaagacca atgcagactt 9240
taatgtcttt tctcttacac taagcaataa agaaataaaa attgaacttc tagtatccta 9300
tttgtttaaa ctgctagctt tacttaactt ttgtgcttca tctatacaaa gctgaaagct 9360
aagtctgcag ccattactaa acatgaaagc aagtaatgat aattttggat ttcaaaaatg 9420
tagggccaga gtttagccag ccagtggtgg tgcttgcctt tatgccttta atcccagcac 9480
tctggaggca gagacaggca gatctctgag tttgagccca gcctggtcta cacatcaagt 9540
tctatctagg atagccagga atacacacag aaaccctgtt ggggaggggg gctctgagat 9600
ttcataaaat tataattgaa gcattcccta atgagccact atggatgtgg ctaaatccgt 9660
ctacctttct gatgagattt gggtattatt ttttctgtct ctgctgttgg ttgggtcttt 9720
tgacactgtg ggctttcttt aaagcctcct tcctgccatg tggtctcttg tttgctacta 9780
acttcccatg gcttaaatgg catggctttt tgccttctaa gggcagctgc tgagatttgc 9840

agcctgattt ccagggtggg gttgggaaat ctttcaaaca ctaaaattgt cctttaattt 9900
tttttttaaa aaatgggtta tataataaac ctcataaaat agttatgagg agtgaggtgg 9960
actaatatta aatgagtccc tcccctataa aagagctatt aaggcttttt gtcttatact 10020
taactttttt tttaaatgtg gtatctttag aaccaagggt cttagagttt tagtatacag 10080
aaactgttgc atcgcttaat cagattttct agtttcaaat ccagagaatc caaattcttc 10140
acagccaaag tcaaattaag aatttctgac ttttaatgtt aatttgctta ctgtgaatat 10200
aaaaatgata gcttttcctg aggcagggtc tcactatgta tctctgcctg atctgcaaca 10260
agatatgtag actaaagttc tgcctgcttt tgtctcctga atactaaggt taaaatgtag 10320
taatactttt ggaacttgca ggtcagattc ttttataggg gacacactaa gggagcttgg 10380
gtgatagttg gtaaaatgtg tttcaagtga tgaaaacttg aattattatc accgcaacct 10440
acttttttaaa aaaaaaagcc aggcctgtta gagcatgctt aaggatcccc taggacttgc 10500
tgagcacaca agagtagtta cttggcaggc tcctggtgag agcatatttc aaaaaacaag 10560
gcagacaacc aagaaactac agttaaggtt acctgtcttt aaaccatctg catatacaca 10620
gggatattaa aatattccaa ataatatttc attcaagttt tcccccatca aattgggaca 10680
tggatttctc cggtgaatag gcagagttgg aaactaaaca aatgttggtt ttgtgatttg 10740
tgaaattgtt ttcaagtgat agttaaagcc catgagatac agaacaaagc tgctatttcg 10800
aggtctcttg gtttatactc agaagcactt ctttgggttt ccctgcacta tcctgatcat 10860
gtgctaggcc taccttaggc tgattgttgt tcaaataaac ttaagtttcc tgtcaggtga 10920
tgtcatatga tttcatatat caaggcaaaa catgttatat atgttaaaca tttgtactta 10980
atgtgaaagt taggtctttg tgggtttgat ttttaatttt caaaacctga gctaaataag 11040
tcatttttac atgtcttaca tttggtggaa ttgtataatt gtggtttgca ggcaagactc 11100
tctgacctag taaccctacc tatagagcac tttgctgggt cacaagtcta ggagtcaagc 11160
atttcacctt gaagttgaga cgttttgtta gtgtatacta gtttatatgt tggaggacat 11220
gtttatccag aagatattca ggactatttt tgactgggct aaggaattga ttctgattag 11280
cactgttagt gagcattgag tggcctttag gcttgaattg gagtcacttg tatatctcaa 11340
ataatgctgg ccttttttaa aaagcccttg ttctttatca ccctgttttc tacataattt 11400
ttgttcaaag aaatacttgt ttggatctcc ttttgacaac aatagcatgt tttcaagcca 11460
tatttttttt cctttttttt tttttttttg gtttttcgag acagggtttc tctgtatagc 11520
cctggctgtc ctggaactca ctttgtagac caggctggcc tcgaactcag aaatccgcct 11580
gcctctgcct cctgagtgcc gggattaaag gcgtgcacca ccacgcctgg ctaagttgga 11640
tatttgttta tataactata accaatacta actccactgg gtggattttt aattcagtca 11700
gtagtcttaa gtggtcttta ttggcccttc attaaaatct actgttcact ctaacagagg 11760
ctgttggtac tagtggcact taagcaactt cctacggata tactagcaga ttaagggtca 11820
gggatagaaa ctagtctagc gttttgtata cctaccagct ttatactacc ttgttctgat 11880
agaaatattt caggacatct agcacccaat tcgccctata gtgagtcgta ttacaattca 11940
ctggccgtcg ttttacaacg tcgtgactgg gaaaaccctg gcgttaccca acttaatcgc 12000
cttgcagcac atcccccttt cgccagctgg cgtaatagcg aagaggcccg caccgatcgc 12060
ccttcccaac agttgcgcag cctgaatggc gaatggacgc gccctgtagc ggcgcattaa 12120
agcgcggcgg gtgtggtggt tacgcgcagc gtgaccgcta cacttgccag cgccctagcg 12180
cccgctcctt tcgctttctt cccttccttt ctcgccacgt tcgccggctt tccccgtcaa 12240
gctctaaatc gggggctccc tttagggttc cgatttagtg ctttacggca cctcgacccc 12300
aaaaaacttg attagggtga tggttcacgt agtgggccat cgccctgata gacggttttt 12360
cgccctttga cgttggagtc cacgttcttt aatagtggac tcttgttcca aactggaaca 12420
acactcaacc ctatctcggt ctattctttt gatttataag ggattttgcc gatttcggcc 12480
tattggttaa aaaatgagct gatttaacaa aaatttaacg cgaattttaa caaaatatta 12540
acgcttacaa tttaggtggc acttttcggg gaaatgtgcg cggaacccct atttgtttat 12600
ttttctaaat acattcaaat atgtatccgc tcatgagaca ataaccctga taaatgcttc 12660
aataatattg aaaaaggaag agtatgagta ttcaacattt ccgtgtcgcc cttattccct 12720
tttttgcggc attttgcctt cctgtttttg ctcacccaga aacgctggtg aaagtaaaag 12780
atgctgaaga tcagttgggt gcacgagtgg gttacatcga actggatctc aacagcggta 12840
agatccttga gagttttcgc cccgaagaac gttttccaat gatgagcact tttaaagttc 12900
tgctatgtgg cgcggtatta tcccgtattg acgccgggca agagcaactc ggtcgccgca 12960
tacactattc tcagaatgac ttggttgagt actcaccagt cacagaaaag catcttacgg 13020
atggcatgac agtaagagaa ttatgcagtg ctgccataac catgagtgat aacactgcgg 13080
ccaacttact tctgacaacg atcggaggac cgaaggagct aaccgctttt ttgcacaaca 13140
tgggggatca tgtaactcgc cttgatcgtt gggaaccgga gctgaatgaa gccataccaa 13200
acgacgagcg tgacaccacg atgcctgtag caatggcaac aacgttgcgc aaactattaa 13260
ctggcgaact acttactcta gcttcccggc aacaattaat agactggatg gaggcggata 13320
aagttgcagg accacttctg cgctcggccc ttccggctgg ctggtttatt gctgataaat 13380
ctggagccgg tgagcgtggg tctcgcggta tcattgcagc actggggcca gatggtaagc 13440
cctcccgtat cgtagttatc tacacgacgg ggagtcaggc aactatggat gaacgaaata 13500

gacagatcgc tgagataggt gcctcactga ttaagcattg gtaactgtca gaccaagttt 13560
actcatatat actttagatt gatttaaaac ttcattttta atttaaaagg atctaggtga 13620
agatcctttt tgataatctc atgaccaaaa tcccttaacg tgagttttcg ttccactgag 13680
cgtcagaccc cgtagaaaag atcaaaggat cttcttgaga tccttttttt ctgcgcgtaa 13740
tctgctgctt gcaaacaaaa aaaccaccgc taccagcggt ggtttgtttg ccggatcaag 13800
agctaccaac tctttttccg aaggtaactg gcttcagcag agcgcagata ccaaatactg 13860
tccttctagt gtagccgtag ttaggccacc acttcaagaa ctctgtagca ccgcctacat 13920
acctcgctct gctaatcctg ttaccagtgg ctgctgccag tggcgataag tcgtgtctta 13980
ccgggttgga ctcaagacga tagttaccgg ataaggcgca gcggtcgggc tgaacggggg 14040
gttcgtgcac acagcccagc ttggagcgaa cgacctacac cgaactgaga tacctacagc 14100
gtgagctatg agaaagcgcc acgcttcccg aagggagaaa ggcggacagg tatccggtaa 14160
gcggcagggt cggaacagga gagcgcacga gggagcttcc agggggaaac gcctggtatc 14220
tttatagtcc tgtcgggttt cgccacctct gacttgagcg tcgatttttg tgatgctcgt 14280
caggggggcg gagcctatgg aaaaacgcca gcaacgcggc ctttttacgg ttcctggcct 14340
tttgctggcc ttttgctcac atgttctttc ctgcgttatc ccctgattct gtggataacc 14400
gtattaccgc c 14411

## Description

### Introduction

The invention provides a method for targeted transgenesis using the Rosa26 locus. Suitable nucleotide acid sequences and vectors for the targeted transgenesis are provided. The Rosa26 locus proved to be a suitable integration site allowing strong and predictable expression of inserted transgenes carrying exogenous promoters.

### Background of the Invention

The generation of transgenic mice by nuclear injection of purified DNA into fertilized eggs is a widely used approach for studying gene or promoter function *in vivo.* However, the level and pattern of expression often varies strongly depending on copy number, configuration, and integration site of the transgene. In addition, founder mice occasionally do not transmit the transgene. Thus, a number of different founders need to be generated and tested in order to identify a useful strain, which is a laborious and time-consuming undertaking (Bradley et. al., Nature Genet., 14:121-123 (1996); Jasin et al., Proc. Natl. Acad. Sci. USA, 93:8804-8808 (1996); Dobie et al., Trends Genet., 13:127-130 (1997); Garrick et al., Nature Genet., 18:56-59 (1998), Al-Shawi et al., Mol. Cell. Boil. 10:1192-1198 (1990)).

To overcome these limitations, homologous recombination in embryonic stem cells has been used to produce mice carrying a single copy of the transgene integrated into a predetermined site of the genome (Shaw-White et al., Transgenic Res.; (1):1-13 (1993); Bronson et al., Proc. Natl. Acad. Sci. USA, 93(17:9067-72 (1996); Hatada et al., J. Biol., Chem., 274(2):948-55 (1999); Vivian et al., Biotechniques, 27(1):154-62 (1999); Evans et al., Physiol. Genomics, Mar. 13, 2(2):67-75 (2000); Cvetkovic et al., J. boil. Chem., 275(2):1073-8 (2000); Guillot et al., Physiol. Genomics, Mar. 13, (2):77-83 (2000); Magness et al., Blood, 95(11):3568-77 (2000); Misra et al., BMC Biotechnol., 1(1):12 (2001); Minami et al., Blood, 100(12):4019-25 (2002); Tang et al., Genesis, 32(3):199-202 (2002)). In these studies, the ubiquitous Hprt locus was more or less successfully used for 'targeted transgenesis'. Insertion of a lacZ gene under the control of the polyoma enhancer/HSV thymidine kinase promoter into the third exon of Hprt resulted in variable β-galactosidase expression that was both orientation and cell-type dependent (Shaw-White et al., Transgenic Res.; (1):1-13 (1993)). Although transgenes under the control of the human and the chicken β-actin gene promoter resulted in widespread expression when inserted into the Hprt locus, the level of transcripts varied strongly in different tissues (Bronson et al., Proc. Natl. Acad. Sci. USA, 93(17:9067-72 (1996)). Unexpectedly, expression of these transgenes, but not of the endogenous Hprt gene appeared to be low or undetectable in kidney and liver (Bronson et al., Proc. Natl. Acad. Sci. USA, 93(17:9067-72 (1996)). Hatada et al. demonstrated that the HPRT locus suppresses the activity of both, the haptoglobin gene promoter as well as the herpes simplex thymidine kinase promoter in several tissues of mice (Hatada et al., J. Biol., Chem., 274(2):948-55 (1999)). Likewise, a human eNOS promoter-LacZ reporter gene placed in the Hprt locus was found to be inactive in hepatic vessels that otherwise express the endogenous eNOS gene (Guillot et al., Physiol. Genomics, Mar. 13, (2):77-83 (2000). Finally, since the HPRT gene is on the X chromosome, transgene expression at this locus is subjected to random X-inactivation. The expression of the transgene in all cells of the female, therefore, requires the generation of homozygotes.

To avoid the complications referred to above, it would be desirable to define an autosomal locus that allows strong and predictable expression of transgenes inserted through homologous recombination. It is, however, not predictable for a person skilled in the art whether chromosomal loci which fulfill these criteria are available at all. Exogenous transgenes may not harbor all of the sequences necessary and sufficient for proper regulation of transcription and may therefore be influenced by cis-regulatory elements near the site of insertion.

The rosa26 locus had been identified by random insertion of retroviral sequences and a β-galactosidase-neomycin resistance fusion gene into the genome of mouse embryonic stem cells (Zambrowicz et al., Proc. Natl. Acad. Sci. USA, 94, 3789-94 (1997)). The rosa26 promoter appeared to mediate ubiquitous expression of promoter-less genes both in embryos and adult mice (Kisseberth et al., Dev. Biol., 214:128-138 (1999); Zambrowicz et al., Proc. Natl. Acad. Sci. USA, 94, 3789-94 (1997)), albeit at different levels in different organs (Vooijs et al., EMBO reports, 21:292-297 (2001)). However, a systematic comparison with other ubiquitous promoters to determine the strength of rosa26 promoter had not been performed. In addition, the activity of exogenous promoters inserted into the rosa26 locus has never been examined.

### Summary of the Invention

The present invention is based on the finding that a particular chromosomal locus present within the eukaryotic genome (including that of mammalian ES cells), namely Rosa26, supports the preservation of the inherent activity of heterologous promoters inserted through homologous recombination at that locus. This chromosomal locus is therefore useful in the context of the "targeted transgenesis" approach for the efficient generation of transgenic organisms (such as mice) with a predictable transgene expression pattern.

Such a "targeted transgenesis" method comprises consecutive experimental steps. A gene expression cassette comprising a suitable promoter (e.g. a ubiquitous or tissue specific promoter, either inducible or constitutive) functionally linked to a gene of interest has to be created; subsequently a vector for the targeted insertion of the above mentioned gene expression cassette into the Rosa26 locus has to be generated; the insertion of the above mentioned gene expression cassette into the Rosa26 locus through homologous recombination in embryonic stem cells follows; finally transgenic mice are generated by the injection of such genetically modified ES cells into blastocysts.

More specifically present invention provides
(1) a method for generating eukaryotic cells having a modified Rosa26 locus, which method comprises the following step (hereinafter shortly referred to as step (a)):
   introducing a functional DNA sequence into the Rosa26 locus of starting eukaryotic cells by homologous recombination which with a targeting vector comprising said functional DNA sequence flanked by DNA sequences homologous to the Rosa26 locus;
(2) the method of (1) above, wherein the eukaryotic cells are mammalian embryonic stem (ES) cells, preferably are non-human mammalian ES cells;
(3) a targeting vector as defined in (1) above;
(4) eukaryotic cells having a modified Rosa26 locus obtainable by the method of (1) and (2) above;
(5) a method for preparing a transgenenic multi-cell organism having a modified Rosa26 locus which comprises utilizing the method as defined in (1) and (2) above;
(6) the method of (5) above, wherein the transgenenic multi-cell organism is a non-human mammal and said method comprises modifying an ES cell as defined in (2) above;
(7) a transgenic multi-cell organism and non-human mammal obtainable by the above defined methods (5) and (6), respectively; and
(8) the use of the eukaryotic cell of (4) above, the transgenic multi-cell organism of (7) above, or the transgenic non-human mammal of (7) above for gene function studies, drug development, as disease model, etc.

The method of the invention offers several advantages over the current technology of pronuclear injection. In particular, the targeting vector allows insertion of a single copy of a gene expression cassette, thus avoiding modulation of transgene expression by the arrangement of multiple copies. By choosing the autosomal Rosa26 locus as insertion site, the expression pattern of the inserted transgene in the non-human animal is predictable; random X-inactivation and/or modulation by chromosomal position effects are avoided. This also eliminates the need to generate and analyse multiple transgenic strains for any given transgene. Finally, the Rosa26 targeting vector for the site-specific integration can be used for multiple gene expression cassettes.

### Description of the Figures

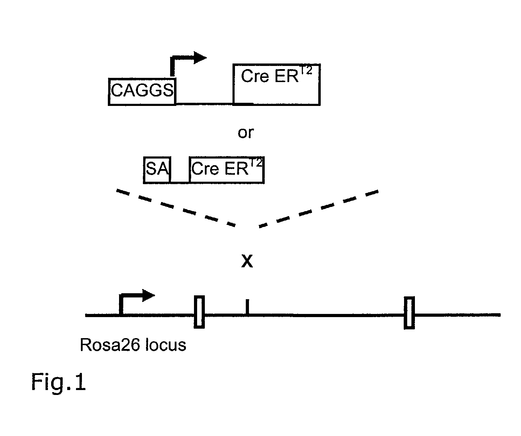

Figure 1: Targeted insertion of CreER and CAGGS-Cre-ER into the Rosa26 locus. A cassette comprising a Cre-ER operationally linked to a CAGGS promoter or a cassette comprising a splice acceptor site (SA) linked to a Cre-ER are inserted into the Rosa26 locus via homologous recombination. A perpendicular dash marks the insertion point within the Rosa26 locus and the rectangular boxes delinate the starting and end points of the Rosa26 transcript.

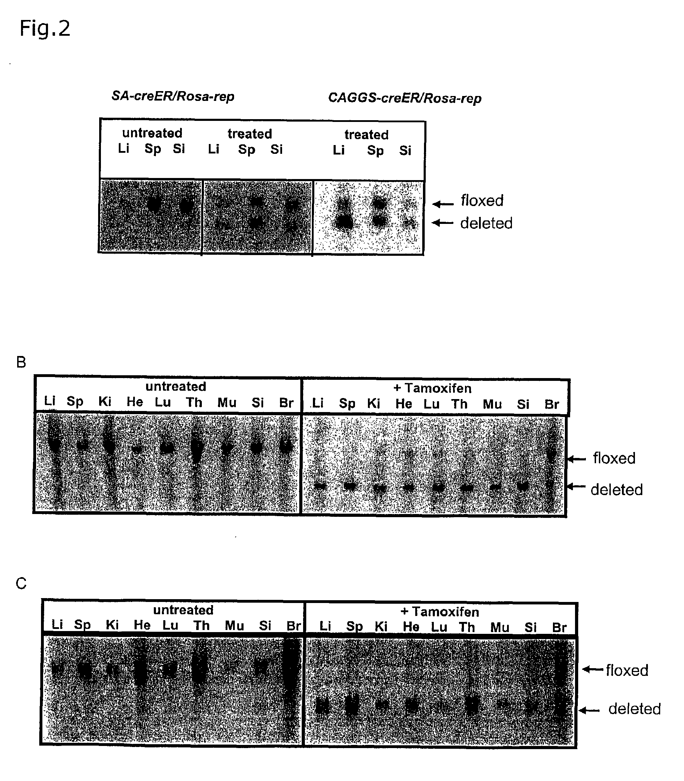

Figure 2: Southern Blot analysis of the inducible recombination of the Rosa (reporter). (A) Genomic DNA was isolated from liver (Li) spleen (Sp) and small intestine (Si) of transgenic mice carrying the SA-creER/Rosa-rep insert or the CAGGS-creER/Rosa-rep insert. To induce the Cre-ER recombinase the mice were treated with Tamoxifen (treated). As a control, a group of mice with the SA-creER/Rosa-rep insert was left untreated (untreated). Presence of the reporter band (floxed) and deletion (deleted) of it upon an induced recombination event are indicated. (B) Transgenic mice carrying at one Rosa26 locus a IoxP flanked DNA polymerase β gene segment (pol^{*βflox*}) and at the other a SA-creER/Rosa-rep were treated with Tamoxifen (treated). A control group of mice was left untreated (untreated). Genomic DNA from liver (Li), spleen (Sp), kidney (Ki). heart (He), lung (Lu), thymus (Th), muscle (Mu), small intestine (Si) and brain (Br) was analysed for presence of pol^{β}^{*flox*}. In a non-recombination event the pol^{*βflox*} band remained (floxed), in a recombination event deletion occurred (deleted). (C) As (B), but mice carried instead of the SA-creER/Rosa-rep the CAGGS-creER/Rosa-rep insert.

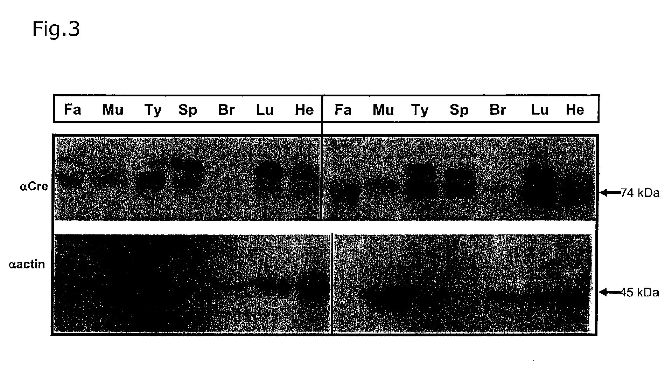

Figure 3: Western Blot analysis of recombinase and α-actin expression. Proteins were extracted from rosa(SA-CreER^{T2}) and rosa (CAGGS-CreER^{T2}) mice and analyzed as described in the "Materials and Method" section. The positions of bands representing CreER^{T2} and actin are indicated. FA: fat tissue, Ty: Thymus; Sp: spleen, Br: Brain, Lu: lung, He: heart.

### Detailed Descripion of the Invention

The term "living organisms" according to the present invention relates to multi-cell organisms which can be vertebrates such as mammals (e.g. non-human animals such as rodents including mice and rats; and humans) or non-mammals (e.g. fish) or can be invertebrates such as insects or worms, or can be plants (higher plants, algi or fungi). Most preferred living organisms are mice and fish.

"Eukaryotic cells" and "starting eukaryotic cells" according to the present invention include cells isolated (derived) from the above defined living organisms and cultured *in* *vitro.* These cells can be transformed (immortalized) or untransformed (directly derived from living organisms; primary cell culture). The term "eukaryotic cells" also includes mono-cellular eukaryotic cells such as yeasts, etc.

It is preferred in the method (1) of the present invention that the eukaryotic cells are derived from a multi-cell organism including vertebrates, invertebrates and plants, preferably is a vertebrate cell, more preferably is derived from a mammal, including rodents such as mouse, rat, etc., or a fish such as zebrafish.

In the methods (1) and (2) of the invention it is preferred that the functional DNA sequence comprises a gene encoding a protein/peptide of interest (i.e. is a expressible and translatable DNA sequence), more preferably said functional DNA sequence is a gene expression cassette (a) comprising a gene of interest operatively linked to a promoter, or (b) is a DNA sequence which can be converted into such gene expression cassette (i.e. into an operatively linked "promoter-gene of interest" construct, e.g. by subsequent modification reactions after its integration). The gene of interest within the gene expression cassette can be any gene coding for a certain protein/peptide of interest, including, but not limited to, recombinases, reporter genes, receptors, signaling molecules, transcription factors, pharmaceutically active proteins and peptides, drug target candidates, disease causing gene products, toxins, etc.

The promoter of the gene expression cassette preferably is a ubiquitous or tissue specific promoter, either constitutive or inducible. Preferred ubiquitous promoter is the CAGGS-promoters are CAGGS, hCMV, PGK; preferred tissue specific promoters are FABP (Saam & Gordon, J. Biol. Chem., 274:38071-38082 (1999)), Lck (Orban et al., Proc. Natl. Acad. Sci. USA, 89:6861-5 (1992)), CamKII (Tsien et al., Cell 87: 1317-1326 (1996)), CD19 (Rickert et al., Nucleic Acids Res. 25:1317-1318 (1997)), Keratin (Li et al., Development, 128:675-88 (201)), Albumin (Postic & Magnuson, Genesis, 26:149-150 (2000)), aP2 (Barlow et al., Nucleic Acids Res., 25 (1997)), Insulin (Ray et al., Int. J. Pancreatol. 25:157-63 (1999)), MCK (Brüning et al., Molecular Cell 2:559-569 (1998)), MyHC (Agak et al., J. Clin. Invest., 100:169-179 (1997), WAP (Utomo et al., Nat. Biotechnol. 17:1091-1096 (1999)), Col2A (Ovchinnikov et al., Genesis, 26:145-146 (2000)); preferred inducible promoter systes are Mx (Kühn et al. Scinence, 269:1427-1429 (1995)), tet (Urlinger et al., Proc. Natl. Acad. Sci. USA, 97:7963-8 (2000)), Trex (Feng and Erikson, Human Gene Therapy, 10:419-27)

It is moreover preferred that the DNA sequences homologous to the Rosa26 locus are 0.2 to 20 kB, preferably 1 to 10 kB long. The targeting vector, functional DNA sequence or gene expression cassette may further comprises one ore more additional functional sequences including but not limited to (selectable) marker genes (such as the neomycin phosphotransferase gene of *E. coli* transposon, etc.), recombinase recognition sites (loxP, FRT, etc.) , poly A signals (such as synthetic polyadenylation sites, or the polyadenylation site of human growth hormones, etc.), splice acceptor sequences (such as a splice acceptor of adenovirus, etc.), introns, tags for protein detection, enhancers, selection markers, etc.

In a particularly preferred embodiment of the method (2) the eukaryotic cells are derived from mouse, the DNA sequences homologous to the Rosa26 locus are derived from the 5' and 3' flanking arm of the mouse Rosa26 locus, preferably said homologous DNA sequences having the sequences shown in SEQ ID NO:4 and 5, respectively, and the promoter is a CAGGS-promoter, most preferably the targeting vector has the sequence shown in SEQ ID NO:7.

The methods (1) and (2) may further (besides step (a) defined above) comprise one or more of the steps (b) isolating the eukaryotic cells, preferably the ES cells having the desired fuctional DNA sequence integrated into the Rosa26 locus; and/or (c) modifying the integrated functional DNA sequence and isolating (ES) cells having the desired modified functional DNA sequence.

The invention also provides a method for preparing a transgenenic multi-cell organism having a modified Rosa26 locus which comprises utilizing the method as defined in (1) and (2) above. This includes a method for preparing a non-human mammal comprising modifying starting ES cells according to steps (a) to (c). The ES cells may subsequently processed according one or more of the following steps: (d) the ES cells obtained in steps (b) or (c) are injected into blastocysts; and/or (e) transgenic non-human animals carrying one or more functional genes of interest at the Rosa26 locus are generated (viz. by well known breeding procedures).

The transgenic multi-cell organisms and non-human mammals obtainable by the method (5) and (6), respectively; preferably have an operatively functional gene expression cassette (as defined above) integrated into its Rosa26 locus. Such transgenic multi-cell organisms and non-human mammals are suitable for gene function studies, drug development, as disease model animals, etc.

The invention is further explained by the following examples and the attached figures, which are, however not to be construed so as to limit the invention.

### Examples

### Materials and Methods

### Plasmid construction:

Rosa-targeting vector: A 129 SV/EV-BAC library (Incyte Genomics) was screened with a probe against *exon2* of the *Rosa26* locus (amplified from mouse genomic DNA using Rscreen1s (GACAGGACAGTGCTTGTTTAAGG) (SEQ ID NO: 1) and Rscreen1as (TGACTACACAATATTGCTCGCAC) (SEQ ID NO:2)). Out of the identified BACclone a 11 kb EcoRV subfragment was inserted into the HindII site of pBS. Two fragments (a 1 kb SacII/XbaI- and a 4 kb XbaI-fragment) were used as homology arms and inserted into a vector consisting of a FRT-flanked neomycin resistance gene (unpublished) and a splice acceptor site from adenovirus (Friedrich G., Soriano P., Genes Dev., 5:1513-23 (1991)). The CAGGS-promoter or the splice acceptor (SA) and a synthetic intron were inserted between the 5' arm and the neomycin resistance gene. The CreER^{T2} and a polyadenylation site (pA) were cloned 3' of the SA and CAGGS-promoter.

Cell culture: Culture and targeted mutagenesis of ES cells were carried out as previously described (Hogan et al., (Cold Spring Harbor Laboratory Press, Cold Spring Harbor NY.), pp. 253-289.) with ES cell lines derived from both inbred and F1 embryos.

Mice: All mice were kept in the animal facility at Artemis Pharmaceuticals GmbH in microisolator cages (Tecniplast Sealsave). B6D2F1 Mice for the generation of tetraploid blastocysts were obtained from Janvier. The *polb*^{*flox*}/*rosa(CreER*^{*T2*}*)* and *ect2*^{*flox*}/*rosa(CreER*^{*T2*}*)* mice were generated by breeding of *rosa(CreER*^{*T2*}*)* ES mice with βT14 (Gu et al., *Science,* 265, 103-106.), respectively.

Production of ES mice by tetraploid embryo complementation: The production of mice by tetraploid embryo complementation was essentially performed as described (Eggan et al., *Proc Natl Acad Sci USA,* 98, 6209-6214.).

Ligand administration: 100 mg Tamoxifen-free base (Sigma, T5648) was suspended in 100 µl Ethanol and solved in 1 ml sunflower oil (Sigma). This 10 mg/100 µl tamoxifen solution was sonicated for 1-2 minutes and then stored at -20°C. For p.o. administration the solution was thawed at 55°C and administrated to 4-8 week old mice by a feeding needle (FST Fine Science Tools GmbH, 18061-20).

Western blot analysis: Western blot analysis was performed using SDS-PAGE (NuPAGE, Invitrogen) and the Breeze Immunodetection System (Invitrogen) according to the manufacturer protocols. Immunodetection was done using sc-543 (HC-20, Santa Cruz Biotechnology, Inc.) against ER, PRB-106C against cre, actin sc-1616 Actin (I-19) against actin and rabbit polyclonal IgG (Santa Cruz Biotechnology, Inc.) antibodies.

### Example 1

A CreER^{T2} gene (Feil et al., (1997) *Biochem Biophys Res Commun.,* 237, 752-757) under the control of the CAGGS-promoter (Buchholz et al. (1996), *Nucl. Acids Res.,* 24, 3118-3119) was inserted into the rosa26 locus by homologous recombination in ES cells (Fig. 1). In addition, the *CreER*^{*T2*} gene was introduced along with a splice acceptor sequence (Friedrich and Soziano (1991), *Genes Dev.,* 9, 1513-1523) as a control for the endogenous activity of the rosa26 gene promoter (Fig. 1). A *loxP*-flanked hygromycin resistance gene was introduced into the second allele of *rosa26* to provide test substrate for Cre *ER*^{*T2*} (Seibler et al., (2003), in press). ES cells modified at both rosa26 alleles were injected into tetraploid blastocysts and completely ES cell derived mice were generated (Eggan et al., (2001). *PNAS,* 98, 6209-6214). *Rosa(SA-CreER*^{*T2*}/*reporter)* and *Rosa(CAGGS-CreER*^{*T2*}/*reporter)* mice were fed with daily 5 mg Tamoxifen for 5 days and recombination of the reporter was analyzed 3 days after the last administration. Southern analysis of genomic DNA from different organs showed up to 50% recombination in the *Rosa(SA-CreER*^{*T2*}/*reporter)* mice and up to 90% recombination in the *rosa(CAGGS-CreER*^{*T2*}/*reporter)* mice, respectively (Fig. 2A). As the second substrate, we used the IoxP flanked DNA *polymerase β* gene segment *(polβ*^{*flox*}*)* (Gu et al., (1994). *Science,* 265, 103-106). The *polβ*^{*flox*}/*rosa(SA-CreER*^{*T2*}*)* and *polβ*^{*flox*}/*rosa(CAGGS-CreER*^{*T2*}*mice* were fed with 5 mg tamoxifen per day for 5 days and analyzed 3 days later. Southern blot analysis revealed that the loxP-flanked *polymerase β* gene segment was excised in more than 90% of cells in all organs except brain in the *rosa(SA-CreER*^{*T2*}/*reporter)* mice (Fig. 2B). In contrast, the degree of inducible recombination was significantly higher in *rosa(CAGGS-CreER*^{*T2*}/*reporter)* mice, reaching 100% efficiency in most organs and up to 70% in brain.

To investigate the pattern and level of *CreER*^{*T2*} expression in *rosa(SA-CreER*^{*T2*}*)* and *rosa(CAGGS- CreER*^{*T2*}*)* mice, we performed Western analysis using antibodies specific for Cre. The 74 kDa band corresponding to the CreER^{T2} fusion protein was detectable in all organs of *rosa(CAGGS- CreER*^{*T2*}*)* mice, including brain (Fig. 3). In contrast, the CreER^{T2} expression level in *rosa(SA-CreER*^{*T2*} mice was significantly lower compared to the *rosa(CAGGS-CreER*^{*T2*}*)* strain and appeared to be undetectable in brain (Fig. 3).

## Claims

1. Method for generating transgenic eukaryotic cells having a modified Rosa26 locus, which method comprises the following step
(a) introducing a functional DNA sequence into the Rosa26 locus of starting eukaryotic cells by homologous recombination with a targeting vector comprising said functional DNA sequence flanked by DNA sequences homologous to the Rosa26 locus.

2. The method of claim 1, wherein the eukaryotic cells
(i) are derived from a multi-cell organism including vertebrates, invertebrates and plants, preferably are vertebrate cells, more preferably are derived from a mammal, including rodents such as mouse, rat, etc., or a fish such as zebrafish; and/or
(ii) are primary cells or immortalized cells;
most preferably the cells are mammalian embryonic stem (ES) cells.

3. The method of claim 1 or 2, wherein
(i) the functional DNA sequence is a gene expression cassette comprising a gene of interest operatively linked to a promoter or is a DNA sequence which can be converted into such gene expression cassette;and/or
(ii) the gene of interest is selected from recombinases, reporter genes, receptors, signaling molecules, transcription factors, pharmaceutically active proteins and peptides, drug target candidates, disease causing gene products, toxins, etc.; and/or
(iii) the promoter is a ubiquitous or tissue specific promoter, either constitutive or inducible, preferably is a CAGGS, hCMV, PGK, FABP, Lck, CamKII, CD19, Keratin, Albumin, aP2, Insulin, MCK, MyHC, WAP, Col2A, Mx, tet or Trex promoter; and/or
(iv) the DNA sequences homologous to the Rosa26 locus are 0.2 to 20 kB, preferably 1 to 10 kB long; and/or
(v) the functional DNA sequence or gene expression cassette further comprises one ore more additional functional sequences including but not limited to marker genes, recombinase recognition sites, poly A signal, introns, etc.; and/or
(vi) the targeting vector further comprises tags for protein detection, enhancers, selection markers, etc.

4. The method of claim 3, wherein the transgenic eukaryotic cells are derived from mouse, the DNA sequences homologous to the Rosa26 locus are derived from the 5' and 3' flanking arm of the mouse Rosa26 locus, preferably said homologous DNA sequences having the sequences shown in SEQ ID NO:4 and 5, respectively, and the promoter is a CAGGS-promoter, most preferably the targeting vector has the sequence shown in SEQ ID NO: 7.

5. The method according to any one of claims 1 to 4, which further comprises one or more of the steps
(b) isolating the eukaryotic cells, preferably the ES cells having the desired functional DNA sequence integrated into the Rosa26 locus; and/or
(c) modifying the integrated functional DNA sequence and isolating ES cells having the desired modified functional DNA sequence.

6. A targeting vector as defined in claims 1 to 4.

7. A eukaryotic cell having a modified Rosa26 locus obtainable by the method of claims 1 to 5.

8. A method for preparing transgenenic multi-cell organism having a modified Rosa26 locus which comprises utilizing the method as defined in claims 1 to 5.

9. The method of claim 8, wherein the transgenenic multi-cell organism is a non-human mammal and said method comprises modifying an ES cell as defined in claims 1 to 5.

10. The method of claim 9 which further comprises one or more of the steps
(d) injecting ES cells obtained in steps (b) or (c) into blastocysts; and/or
(e) generating transgenic non-human animals carrying one or more functional genes of interest at the Rosa26 locus.

11. A transgenic multi-cell organism and a transgenic non-human mammal obtainable by the method of claim 8 and 9-10, respectively, and having an operatively functional gene expression cassette integrated into its Rosa26 locus.

12. Use of the eukaryotic cell of claim 7, the transgenic multi-cell organism of claim 11, or the transgenic non-human mammal of claim 11 for gene function studies, drug development, as disease model animals, etc.
